(19) Europäisches Patentamt
European Patent Office
Office européen des brevets

(11) Publication number: **0 275 104 B1**

# (12) EUROPEAN PATENT SPECIFICATION

(45) Date of publication of patent specification: **19.01.94**

(51) Int. Cl.5: **C07D 233/60**, C07F 7/18, C07C 69/757, C07C 62/32, C07C 62/34

(21) Application number: **88100467.5**

(22) Date of filing: **14.01.88**

The file contains technical information submitted after the application was filed and not included in this specification

(54) **Process for preparing 2,3-dihydro-1H-imidazolylalkoxyindene derivatives and pharmaceutically acceptable salts, and intermediate therefor.**

(30) Priority: **14.01.87 ES 8700155**

(43) Date of publication of application:
**20.07.88 Bulletin 88/29**

(45) Publication of the grant of the patent:
**19.01.94 Bulletin 94/03**

(84) Designated Contracting States:
**BE CH DE FR GB IT LI NL**

(56) References cited:
**EP-A- 0 021 368**

**CHEMICAL ABSTRACTS, vol. 106, no. 3, 19 January 1987, Columbus, OH (US); R. FOOUET AMBROS et al., p. 607, no. 18549c&NUM;**

**CHEMICAL ABSTRACTS, vol. 106, no. 5, 02 February 1987, Columbus, OH (US); R. FOOUET AMBROS et al., p. 530, no. 32847e&NUM;**

(73) Proprietor: **FERRER INTERNACIONAL, S.A.**
**Gran Via Carlos III, 94**
**08028 Barcelona(ES)**

(72) Inventor: **Foguet, Rafael**
**Llusanés 8**
**E-08022 Barcelona(ES)**
Inventor: **Forné, Ernesto**
**Felipe de Paz 15**
**E-08028 Barcelona(ES)**
Inventor: **Sacristán, Aurelio**
**Santa Amelia 2**
**E-08034 Barcelona(ES)**
Inventor: **Castell , Josep M.**
**Princep d'Asturias 35**
**E-08012 Barcelona(ES)**
Inventor: **Ortiz, José A.**
**C rcega 429**
**E-08037 Barcelona(ES)**

(74) Representative: **Kinzebach, Werner, Dr. et al**
**Patentanwälte**
**Reitstötter, Kinzebach und Partner**
**Sternwartstrasse 4**
**Postfach 86 06 49**
**D-81633 München (DE)**

EP 0 275 104 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for preparing 2,3-dihydro-1H-imidazolylalxoxyindene derivatives having the formula (I):

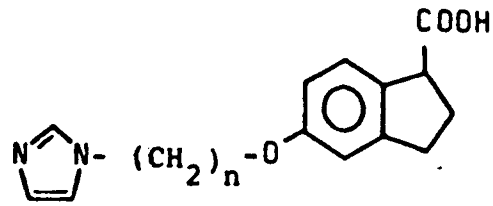

(I)

wherein n is 1 to 4 and preferably 2,
and its pharmaceutically acceptable salts. The 2,3-dihydro-1H-imidazolylalkoxyindene derivatives which are useful as antithrombotic agents and a process for preparing such compounds are disclosed and described in EP-A-0 184 809.

The preferred compound of formula (I) is 2,3-dihydro-5-[β-(1H-imidazol-1-yl(ethoxy]-1H-indene-1-carboxylic acid and the preferred pharmaceutically acceptable salt is hydrochloride.

Further, the invention relates to the intermediates of formula III:

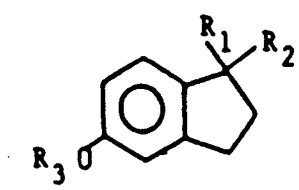

(III)

wherein

a) $R_1$ is cyano; $R_2$ is trimethylsilyloxy or hydroxyl and $R_3$ is methyl;

b) $R_1$ is COOH;

$R_2$ is H and $R_3$ is hydrogen, methyl or $X(CH_2)_n$-in which n is 1 to 4 and X is chloro, bromo, iodo or $OSO_2R'_2$ in which $R_2$' is phenyl, p-methylphenyl or methyl; or

c) $R_1$ is COOR, in which R is $C_1$ - $C_4$ alkyl,

$R_2$ is H and $R_3$ is hydrogen, methyl or $X(CH_2)_n$-, n is 1 to 4 and X is chloro, bromo, iodo, imidazole-1-yl or $OSO_2R_2$' and $R_2$' is phenyl, p-methylphenyl or methyl

The inventors have found out that the compounds of formula (I) can be advantageously obtained by a new process different from that disclosed in the aforesaid European patent application. In effect, the process of this invention allows to obtain the intermediates in sufficient purity and high yield and to use them without further purification. Thus the new process is more efficient and in particular suitable in the preparation of large quantities, higher than 500 g of the compounds of formula (I). The process of the invention is illustrated by means of the preparation of 2,3-dihydro-5-[β-(1H-imidazol-1-yl)ethoxy] -1H-indene-1-carboxylic acid hydrochloride according to the following scheme:

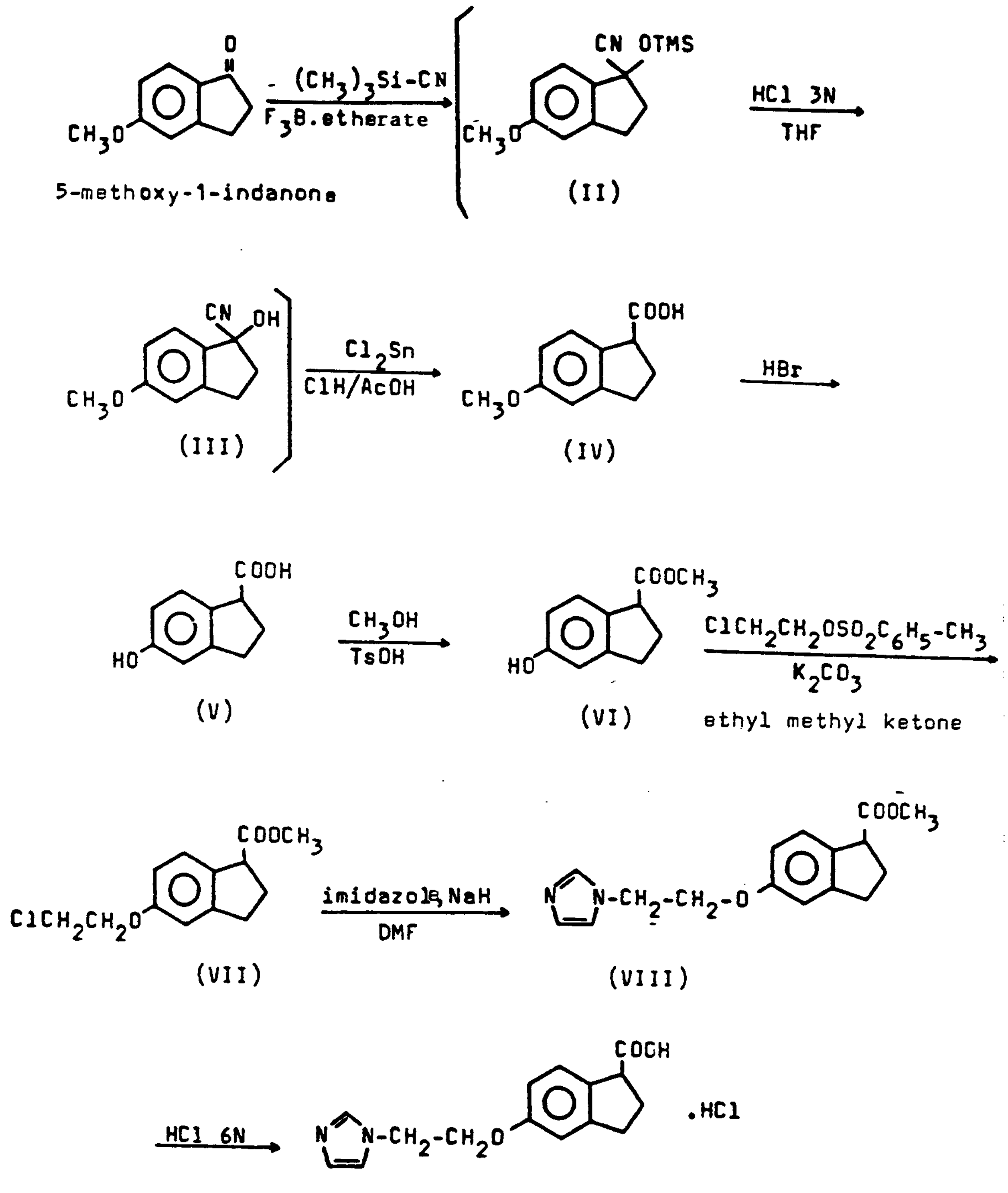


5-methoxy-1-indanone (II) (III) (IV) (V) (VI) (VII) (VIII) (I)

All the intermediates used are new and will also be subject matter of the present invention.

The inventors have found out that 5-methoxy-1-indanone-cyanohydrin may be prepared by reaction with trimethylsilylcyanide in the presence of a Lewis acid as a catalyst. Preferred catalysts are boron trifluoride etherate, zinc iodide and aluminium trichloride. The reaction is carried out at elevated temperature, preferably at 70 to 120 °C.

The acidic hydrolysis of 2,3-dihydro-5-methoxy-O-(trimethylsilyl)-1H-inden-1-one-cyanohydrin leads to 2,3-dihydro-5-methoxy-1H-inden-1-one-cyanohydrin which, like its precursor, does not require any purification to proceed with the process. Preferably a mineral acid (e.g. $H_2SO_4$, $H_3PO_4$ and preferably HCl) is used. The reaction medium is preferably an ether (e.g. tetrahydrofurane or dioxane), the reaction temperature preferably ranges from 40 °C to the boiling temperature of the mixture.

The cyanohydrin is converted into 2,3-dihydro-5-methoxy-1H-inden-1-carboxylic acid by using stannous chloride in an acidic medium, preferably concentrated hydrochloric acid and glacial acetic acid. The

reaction temperature may range from ambient temperature to the boiling temperature of the reaction medium, preferably from 80 to 110 °C.

Demethylation of 2,3-dihydro-5-methoxy-1H-inden-1-carboxylic acid may be effected in a strong acidic medium, preferably with hydrobromic acid. The reaction temperature preferably ranges from 130 °C to the boiling point of the reaction mixture. 2,3-Dihydro-5-hydroxy-1H-inden-1-carboxylic acid is obtained which, by reacting with an aliphatic $C_1$-$C_4$ alcohol in the presence of an acid as catalyst, preferably a sulphonic acid, leads to the corresponding ester. A preferred aliphatic alcohol is methanol. p-Toluenesulphonic acid is preferably used as a catalyst in this esterification. The reaction occurs at elevated temperatures, preferably at the boiling point of the mixture. Thus, methyl 2,3-dihydro-5-hydroxy-1H-inden-1-carboxylate is obtained.

The aforesaid ester is reacted with an alkylating agent of the general formula X-$(CH_2)_n$-Y wherein X and Y independently may be chloro, bromo, iodo or $OSO_2R_2'$ in which $R'$ is phenyl, p-methylphenyl or methyl. In the preferred alkylating agent X is chloro and Y is $OSO_2R_2'$ with $R_2'$ being p-methylphenyl particularly preferred. The reaction is carried out in a non-polar medium which preferably is an aliphatic keton, in particular methylethyl keton.

Further, the reaction is carried out in the presence of a base which is preferably selected from alkaline or alkaline earth carbonates or bicarbonates, such as sodium and calcium carbonate and in particular potassium carbonate.

The reaction occurs from room temperature to the boiling point of the reaction medium, but is preferably conducted at the boiling point of the reaction medium.

Then, imidazole is alkylated with the so obtained ester of the general formula VII′ in a suitable solvent, such as an N,N-substituted linear amide. Dimethylformamide is the preferred solvent. The reaction is carried out in the presence of a base, such as alkaline hydrides, preferably sodium hydride. The reaction temperature may range from room temperature to the boiling point of the mixture preferably from 80° to the boiling point.

The so obtained ester of the general formula VIII′ is then hydrolyzed, for example with a mineral acid, preferably hydrochloric acid, or with diluted inorganic bases, such as sodium hydroxide. Preferably, the reaction is carried out at the boiling temperature of the mixture. If the hydrolysis is effected with an acid the compounds of the general formula I will be obtained in the form of an acid addition salt.

EXAMPLE 1: 2,3-Dihydro-5-methoxy-1H-indene

In a 250-litre stainless steel reactor (Pilot Plant) 6.81kg of 97% sodium hydroxyde in 100 l of distilled water were dissolved, and under heating at 170°C, 22 kg of 2,3-dihydro-1-H-inden(5-indanol) were added in the course of 20 min and then stirred to total dissolution, 20.13 kg of freshly distilled dimethyl sulphate were added through a funnel for about 1 hour. Then the mixture was refluxed for 8 hours and allowed to cool. The upper organic phase was decanted, the aqueous phase was extracted twice with 5 l of toluene, the organic phases were washed with 10% sodium hydroxyde solution and with water and the solvent was distilled under vacuum to give 18.7 kg (77 %) of a colourless liquid. GLC 99% $n_D^{20}$ = 1.5430; IR and $^1$H-NMR spectra were consistent with structure.

$^1$H-NMR Spectrum ($CDCl_3$), ppm: 2.1 (m, 2H; $-CH_2-$), 3.85 (m, 4H; Ar $>$$(CH_2)_2$-), 3.74 (s, 3H; $-OCH_3$), 6.55-6.88 (m, 2H;Ar-) and 7.08 (d, 1H, $J_o$ = 8Hz; Ar-).

EXAMPLE 2: 5-methoxy-indanone

In a 250-litre stainless steel reactor (Pilot Plant) containing 9.45 kg of 2,3-dihydro-5-methoxy-1H-indene in 54 l of glacial acetic acid cooled at 5-10ºC, 10.08 kg of chromic anhydride (1.58 equivalents) dissolved in 5 l of water and 27.5 l of glacial acetic acid are added during 4.5-5 hours at 5-10ºC. Then, the mixture was kept at 5°C for further 5 hours and at room temperature overnight under stirring. The reaction mixture was poured, under vigorous stirring, onto 800 l of water cooled to 5°C and 5-methoxy-1-indanone precipitated (as supernatant). The precipitate was filtered by centrifugation, washed with 30 l of water, centrifugated again and dried to constant weight to give 6.83 kg (66%) of a yellow solid. M.p. 108-110ºC; GLC 98.9%; water content (KF): lower than 0.1%; IR and $^1$H-NMR spectra were consistent with structure.

IR Spectrum (KBr), $cm^{-1}$: 3060-2840, 1690, 1600, 1250, 1095, 840.

$^1$H-NMR Spectrum ($CDCl_3$),ppm: 2.5-2.75 (m,2H;$-CH_2-$), 2.9-3.2 (m,2H;$-CH_2-$); 3.85(s,3H;$-OCH_3$), 6.88-(m,2H;Ar-) and 7.67 (d,1H,$J_o$= 8Hz; Ar-).

EXAMPLE 3: 2,3-Dihydro-5-methoxy-O-(trimethylsilyl)-1H-inden-1-one cyanohydrin (II)

2.228 kg of 5-methoxy-1-indanone were introduced into a 20-litre reactor provided with a mechanical stirrer, addition funnel, thermometer and cooling tower which outlet was successively connected to a tower drier and a gas absorption tower loaded with sodium hydroxyde solution. The system was subjected to nitrogen drain and when stabilized in this manner, 1.5 kg of trimethylsilylcyanide (1.1 equivalents) and 15 ml of boron trifloride etherate were rapidly poured through the funnel. Then, the reactor was immersed in a previously-heated oil bath (80ºC). After 45 minutes, the reaction mass started to soften and stirring was started; the temperature was kept at 75-80ºC for 2 hours and at 100ºC for further 2 hours. After overnight at room temperature under nitrogen atmosphere, the resulting oily liquid was characterized by respective IR and $^1$H-NMR spectra.

IR Spectrum (film), $cm^{-1}$: 3020-2840, 2730 (weak), 1465, 1250, 1080, 875, 840.

$^1$H-NMR Spectrum ($CDCl_3$), ppm: 0.27 (s, 9H; $Si(CH_3)_3$), 2.0-3.0 (multiple bands, 4H; $(-CH_2)_2$-), 3.55 (s, 3H; $CH_3O$-), 6.38-6.88 (m, 2H; Ar-) and 7.22 (d, $J_o$ = 8Hz, 1H; Ar-).

EXAMPLE 4: 2,3-Dihydro-5-methoxy-1H-inden-1-one-cyanohydrin (III)

Into the reactor containing the reaction product of Ex. 3, 3.7 l of tetrahydrofurane and through the funnel 3375 ml of 3N hydrochloric acid were added, a slight exothermia occurred till reaching 40ºC. When the exothermia became stabilized, the reaction mixture was heated in an oil bath at 66-70ºC for 1 hour, then it was allowed to cool at room temperature; 3375 of water were added, the organic phase was decanted and the aqueous phase was extracted twice with 3 l of toluene. The combined organic extracts were concentrated to dryness at vacuum. The oily residue was solidified under vacuum.

IR Spectrum (film), $cm^{-1}$: 3420, 3020-2820, 2220 (intense), 1475, 1295, 1250, 1110, 1015, 840, 810.

$^1$H-NMR Spectrum ($CDCl_3$), ppm: 2.4-3.1 (wide, 4H;$(-CH_2)_2$-), 3,50 (s, 1H; -OH), 3.80 (s, 1H; $CH_3O$-), 6.6-7.15 (multiple bands, 2H; Ar-) and 7.37 (d, 1H, $J_o$ = 8Hz; Ar-).

EXAMPLE 5: 2,3-Dihydro-5-methoxy-1H-inden-1-carboxylic-acid (IV)

In a 100-litre glass reactor (Pilot Plant) connected to a gas absorption tower containing sodium hydroxyde solution, 16.3 l of concentrated hydrochloric acid, 7.14 kg of stannous chloride dihydrate and 14 l of glacial acetic acid were added under heating till the reaction mass reached 90ºC. Then, 2.3 kg of 2,3-dihydro-5-methoxy-1H-inden-1-one cyanohydrin dissolved in 10 l of glacial acetic acid were added and heated at 100-105ºC for 10 hours. The mixture was allowed to cool to 50°C, 20 l of toluene and 30 l of water were added and stirred for 1 hour. The aqueous phase was decanted, the insoluble material was filtered and after washing with toluene the residue was discarded; then the aqueous phase was extracted twice with 18 l of toluene and filtered if necessary. The filtered organic extracts were washed twice with 35 l of saturated sodium chloride solution and once with water, then evaporated to dryness at vacuum; the residue, as brown semisolid, was a mixture of carboxylic acid and ketone weighing 2.3 kg. It was taken up in 25 l of toluene and 15 l of 1N potassium hydroxyde solution and stirred for 30 minutes; the toluene phase was extracted for the second time with 15 l of 1N sodium hydroxyde solution. The alkaline extracts, cooled at 10ºC, and 4 l of methylene chloride were acidified with 6N hydrochloric acid, further 6 l of methylene chloride were added and the organic phase was separated; the aqueous phase was extracted twice with 10 l of methylene chloride, and the organic extracts were washed several times with saturated sodium chloide solution (diluted with water), dried over magnesium sulphate and concentrated to dryness at vacuum. The residue as brown solid weighed 1.6 kg (69%), m.p. 111-116ºC, acid group titration: 96%, and was crystallized from acetonitrile, m.p. 118-121ºC.

IR Spectrum (KBr), $cm^{-1}$: 3300-2400, 1700, 1480, 1420, 1315, 1260, 1170, 1140, 1090, 1030, 860, 790.

$^1$H-NMR Spectrum ($CDCl_3$), ppm: 2.2-3.2 (2 multiplets, 4H; $-(CH_2)_2$-), 3.74 (s, 1H; $CH_3O$-), 3.98 (t, 1H, J = 7Hz; $>$CH-), 6.6-6.87 (multiple bands, 2H; Ar-) and 7.27 (d, 1H, $J_o$ = 8Hz;Ar-).

EXAMPLE 6: 2.3-Dihydro-5-hydroxy-1H-inden-1-carboxylic acid (V)

In a 20-litre reactor provided with a mechanical stirrer, 1.6 kg of 2,3-dihydro-5-methoxy-1H-inden-1-carboxylic acid and 8 l of 48% hydrobromic acid were introduced and heated at 135-140ºC in oil bath for 15 hours. The mixture was allowed to cool, and the hydrobromic acid was evaporated at vacuum; to the blackish residue, 3 l of toluene were added and the solvent was evaporated at vacuum. This operation was ride again with further 3 l of toluene to give the desired compound as blackish solid, 1.5 kg (100%), m.p.

137-138ºC and acid group titration: 101%.

IR Spectrum (KBr), $cm^{-1}$ : 3600-2600, 1710, 1610, 1500, 1460, 1230, 1180, 855, 820, 755.

$^1$H-NMR Spectrum ($d_6$-DMSO), ppm: 2.0-3.0 (2 multiplets, 4H; $-(CH_2)_2-$), 3.81 (t, 1H, J = 7Hz; >CH-), 6.5-6.75 (m, 2H; Ar-), 7.08 (d, 1H, $J_o$ = 8Hz; Ar-) and 8.75 (wide, 2H; -OH and -COOH).

EXAMPLE 7: Methyl 2,3-dihydro-5-hydroxy-1H-inden-1-carboxylate (VI)

In a 20-litre reactor with a mechanical stirrer, 1.6 kg of 2,3-dihydro-5-hydroxy-1H-inden-1-carboxylic acid were dissolved with 7.2 l of absolute methanol, 9 g of p-toluen esulphonic acid were added and then refluxed under stirring in an oil bath heated at 90ºC for 24 hours. The mixture was allowed to cool, the excess of methanol was evaporated at vacuum, the residue was diluted with 1.5 l of water, 1.5 l of saturated sodium bicarbonate solution and 5 l of toluene and then stirred for 30 minutes. The insoluble material was filtered, washed twice with 1 l of toluene; the aqueous phase was extracted with 2 l of toluene, and the organic phases were filtered using a filter paper and washed with water. The toluene phase was concentrated at vacuum to give a blackish, viscous liquid residue weighing 1.391 kg (88%), which was distilled at vacuum to give 1.1 kg (70%) of colourless liquid at 124-137ºC/0.03-0.025 tors, $n_D^{20}$ = 1.5533, IR and $^1$H-NMR spectra were consistent with structure.

IR Spectrum (film), $cm^{-1}$: 3400, 3040-2840, 1710, 1490, 1450, 1340, 1260, 1210, 1170, 850, 810.

$^1$H-NMR Spectrum ($CDCl_3$), ppm: 2.1-3.1 (2 multiplets, 4H; $-(CH_2)_2-$), 3.70 (s, 3H; $-COOCH_3$), 3.96 (t, 1H; J = 6.5 Hz; >CH-), 5.95 (s, 1H; -OH), 6.5-6.75 (m, 2H; Ar-) and 7.15 (d, 1H, $J_o$ = 8Hz; Ar-).

EXAMPLE 8: Methyl 2,3-dihydro-5-(β-chloroethoxy)-1H-inden-1-carboxylate (VII)

In a 20-litre reactor provided with a mechanical stirrer and reflux cooler, 1078 g of methyl 2,3-dihydro-5-hydroxy-1H-inden-1-carboxylate were dissolved in 9 l of dry methyl ethyl ketone, 1162 g of anhydrous potassium carbonate were added and stirred for 30 minutes; then 1448 g of 2-chloroethyl p-toluene sulphonate were added and refluxed in an oil bath for 50 hours. The mixture was allowed to cool, the insoluble solid was filtered and washed twice with acetone over the same filter. The filtrate was evaporated and dried at vacuum, the residue was dissolved in 4.7 l of toluene and extracted with 1.8 l of 3N sodium hydroxyde solution in an decantation funnel with mechanical stirring for 15 minutes; then it was neutralized with water and dried. After evaporation of the solvent at vacuum, the residue as reddish liquid weighed 1419 g (99%). Thin-layer chromatography (silica-gel) showed a major spot and a slight shadow at lower Rf. Cl analysis correct. It was used for the following synthesis step without further purification.

IR Spectrum (film), $cm^{-1}$: 3020-2860, 1730, 1610, 1490, 1435, 1270, 1170, 1040, 810.

$^1$H-NMR Spectrum ($CDCl_3$), ppm: 2.2-3.2 (2 multiplets, 4H; $-(CH_2)_2$), 3.68 (s + t, 5H; $-COOCH_3$ and $-CH_2Cl$), 3.98 (t, 1H, J = 7Hz; >CH-), 4.19 (t, 2H, J = 6Hz; $-OCH_2-$), 6.65-6.8 (m, 2H; Ar-) and 7.26 (d, 1H, $J_o$ = 8Hz; Ar-).

EXAMPLE 9: Methyl 2,3-dihydro-5-[β-(1H-imidazol-1-yl)ethoxy]-1H-inden-1-carboxylate (VIII)

In a 20-litre reactor provided with a mechanical stirrer, reflux cooler, thermometer, compensatory pressure funnel and nitrogen atmosphere, 183.84 g of 80% sodium hydride (20% paraffin) and 2.8 l of dry dimethylformamide (4 Å sieves) were dissolved. Then a solution of 417.19 g of imidazole in 1.5 l of DMF was added under stirring and cooling at 10-20ºC ($t_R$) in water-ice bath; after completion of the addition, the bath was withdrawn and the mixture was heated at 90-100ºC for 30 minutes using an electrical blanket until the hydrogen liberation and the formation of the anion was complete. After cooling of the mixture again to room temperature, a solution of methyl 2,3-dihydro-5-(β-chloro-ethoxy)-1H-inden-1-carboxylate in 4 l of DMF was added and then heated at 90-100ºC ($t_R$) for 9 hours. After cooling, the mixture was poured to 10 l of water and 10 kg of ice under stirring and extracted twice with 5 l of methylene chloride, and the organic phases were washed with water (4 times) and neutralized. The desired compound was successively extracted with 3.2 l and 1 l of 2N hydrochloric acid and 1 l of water; the aqueous extracts were washed with 2.5 l of methylene chloride and taken to basic pH by adding 1 l of ammonium hydroxide concentrated solution under cooling. The precipitate was extracted with 3 l and 1.5 l of methylene chloride and the organic extracts were washed (3 times) with 3 l of water, dried and concentrated to dryness. The residue, as brownish oil, weighed 1264 g (79%) and thin-layer chromatography (silica-gel) showed a major spot spectroscopic data; it was used for the following synthesis step without further purification.

IR Spectrum (film), $cm^{-1}$: 3100-2840, 1730, 1490, 1430, 1250, 1170, 1070, 815.

$^1$H-NMR Spectrum ($CDCl_3$), ppm: 2.2-3.2 (2 multiplets, 4H; $-(CH_2)_2-$), 3.67 (s, 3H; $-COOCH_3$), 3.96 (t, 1H, J = 7Hz; >CH-), 4.20 (m, $A_2B_2$ system, 4H; $-CH_2-CH_2-$), 6.6-7.4 (multiple bands, 5H; Ar-, imidazole) and 7.55 (s, 1H; imidazole).

EXAMPLE 10: 2,3-Dihydro-5-[β-(1H-imidazol-1-yl)ethoxy]-1H-inden-1-carboxylic acid hydrochloride

In a 5-litre reactor provided with a mechanical stirrer and cooler, a mixture of 1264 g of methyl 2,3-dihydro-5-[β-(1H-imidazol-1-yl)ethoxy]-1H-inden-1-carboxylate and 3.5 l of 6N hydrochloric acid was refluxed, and the formed methanol ($t_R$ = 100-107ºC) was removed. The mixture was allowed to cool and the hydrochloric acid was evaporated at vacuum. When stirring of the resulting mass became rather difficult, 0.8 l of benzene were added twice under vacuum distillation so as to facilitate the hydrochloric acid removal. In the same manner, 1.8 l of acetone were added and distilled till the mass started to crystallize, then further 1.8 l of acetone were added and distillation was continued to a residual volume of 2.5-3 l, and thus the mass was crystallized for the most part. After standing overnight, 1.2 l of acetone were added and refluxed for 20 minutes under stirring. The resulting yellowish solid was cooled with water-ice to 5ºC, filtered, washed with the minimal quantity of cold acetone (1,5 l) and dried at 50 °C in a vacuum stove under potassium-hydroxide/phosphorous pentoxide to constant weight: 1200 g (88%) of a white-yellowish solid were obtained, m.p. 170-173ºC which was recrystallized from 12 l of isopropanol under reflux; 145 ml of water and 55 g of active carbon were added. After cooling, filtering off and drying to constant weight, 981 g (72%) of a white solid were obtained, m.p. 171-173ºC. Analysis correct.

IR Spectrum (KBr), $cm^{-1}$: 3300-2400, 1680, 1480, 1266, 1220, 1080, 1040, 820, 735.

$^1$H-NMR Spectrum ($d_4$-MeOH), ppm: 2.3 and 2.9 (2m, $A_2B_2$ system; $Ar-CH_2-CH_2-$), 3.9 (t, 1H, J = 7.5Hz; >CH-OOH), 4.32 and 4.67 (2m, $A_2$ and $B_2$ system; $-CH_2-CH_2O-$), 6.6-6.9 (m, 2H; Ar-), 7.24 (d, 1H; $J_o$ = 8.1Hz; Ar-), 7.56 and 7.74 (2d, 2H; J = 1.4Hz; imidazole) and 5.07 (d, 1H, J = 1.4Hz; imidazole).

## Claims

**1.** A process for preparing 2,3-dihydro-1H-imidazolylalkoxy-indene derivatives having the formula (I):

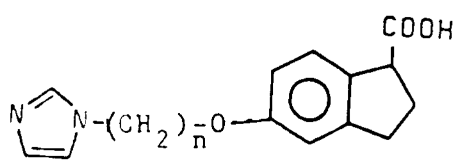

(I)

wherein n is 1 to 4
and the pharmaceutically acceptable salts thereof.
**characterized in that**

a) 5-methoxy-1-indanone is reacted with trimethylsilylcyanide in the presence of a Lewis acid as a catalyst at an elevated temperature, thus obtaining the O-trimethylsilyl cyanohydrine having the formula (II):

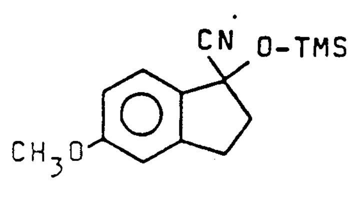

(II)

b) the O-trimethylsilyl cyanohydrine of the said formula (II) is hydrolyzed with an acid in an inert medium, thus obtaining the cyanohydrine having the formula (III):

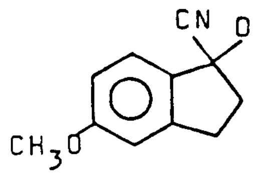

(III)

c) the cyanohydrine of the said formula (III) is treated with stannous chloride in an acidic medium thus obtaining the acid having the formula (IV):

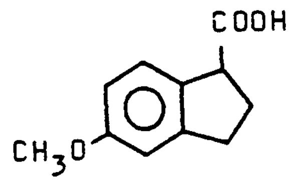

(IV)

d) the acid of the said formula (IV) is demethylated, thus obtaining the acid of formula (V):

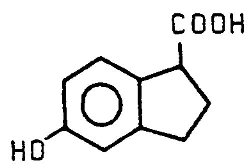

(V)

e) the acid of the said formula (V) is esterified with an aliphatic alcohol, having up to 4 carbon atoms of the general formula ROH (V'), in the presence of an acid as a catalyst, thus obtaining an ester of general formula (VI'):

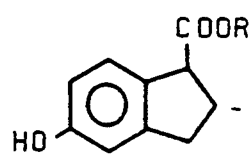

(VI')

wherein R is as defined above;

f) an ester of the said general formula (VI') is alkylated with $X-(CH_2)_n-Y$, wherein X and Y independently may be chloro, bromo, iodo or $OSO_2R_2'$ in which $R_2'$ is phenyl, p-methylphenyl or methyl, in the presence of a base, and in a non-polar medium thus obtaining an ester of general formula (VII'):

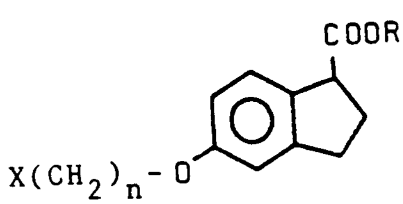

(VII')

wherein R, X and n are as defined above;

g) an ester of the said general formula (VII') is reacted with imidazole in solution of a N,N-substituted linear amide and in the presence of a basic coadjuvant, thus obtaining an ester of general formula (VIII'):

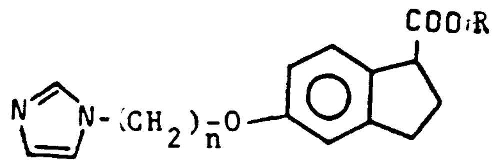

VIII'

wherein R and n are as defined above;
h) an ester of the said general formula (VIII') is hydrolyzed.

**2.** A process as claimed in Claim 1, wherein said Lewis acid in step a) is boron trifluoride etherate.

**3.** A process as claimed in Claims 1 or 2, wherein said acid in step b) is a mineral acid, preferably hydrochloric acid.

**4.** A process as claimed in anyone of the preceding claims, wherein said inert medium in step b) is an ethereal medium, preferably tetrahydrofurane.

**5.** A process as claimed in anyone of the preceding claims, wherein said acidic medium in step c) is a mixture of concentrated hydrochloric acid and glacial acetic acid.

**6.** A process as claimed in anyone of the preceding claims, wherein step d) is carried out in a strong acid medium, preferably in concentrated hydrobromic acid.

**7.** A process as claimed in anyone of the preceding claims, wherein the catalyst in step e) is a sulphonic acid, preferably p-toluenesulphonic acid.

**8.** A process as claimed in anyone of the preceding claims, wherein in step f) X is chloro and Y is $OSO_2R''$, preferably p-toluenesulphonyl.

**9.** A process as claimed in anyone of the preceding claims, wherein said base in step f) is an alkaline or alkaline earth carbonate or bicarbonate, preferably potassium carbonate.

**10.** A process as claimed in anyone of the preceding claims, wherein said non polar medium in step f) is an aliphatic ketone, preferably methylethylketone.

**11.** A process as claimed in anyone of the preceding claims, wherein said N,N-disubstituted linear amide in step g) is dimethylformamide.

**12.** A process as claimed in anyone of the preceding claims, wherein said basic co-adjuvant in step g) is sodium hydride.

**13.** A process as claimed in anyone of the preceding claims, wherein said mineral acid in step h) is hydrochloric acid.

**14.** A process as claimed in Claim 2, wherein R in the general formulae V' , VI', VII' and VIII' is methyl.

**15.** A process as claimed in anyone of the preceding claims, wherein a compound of the general formula (I) is obtained in which n is 2.

**16.** The compounds of the general formula:

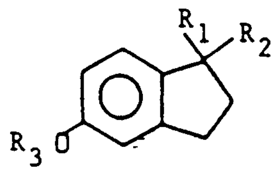

(III)

wherein

a) $R_1$ is cyano; $R_2$ is trimethylsilyloxy or hydroxyl and $R_3$ is methyl;

b) $R_1$ is COOH;

$R_2$ is H and $R_3$ is hydrogen, methyl or $X(CH_2)_n$- in which n is 1 to 4 and X is chloro, bromo, iodo or $OSO_2R'_2$ in which $R_2'$ is phenyl, p-methylphenyl or methyl; or

c) $R_1$ is COOR, in which R is $C_1$ - $C_4$ alkyl,

$R_2$ is H and $R_3$ is hydrogen, methyl or $X(CH_2)_n$-, n is 1 to 4 and X is chloro, bromo, iodo, imidazol-1-yl or $OSO_2R_2'$ and $R_2'$ is phenyl, p-methylphenyl or methyl.

## Patentansprüche

**1.** Verfahren zur Herstellung von 2,3-Dihydro-1H-imidazolylalkoxyindenderivaten der Formel (I):

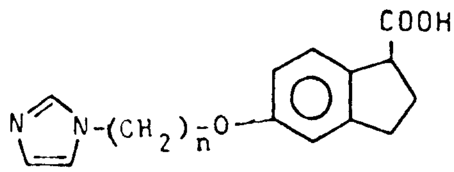

(I)

worin n für 1 bis 4 steht, und der pharmazeutisch akzeptablen Salze davon, dadurch gekennzeichnet, daß man

a) 5-Methoxy-1-indanon mit Trimethylsilylcyanid in Anwesenheit einer Lewissäure als Katalysator bei erhöhter Temperatur zur Reaktion bringt, wobei man das O-Trimethylsilylcyanohydrin der Formel (II):

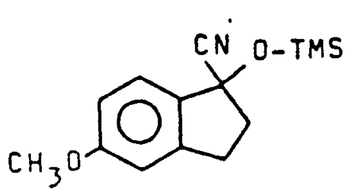

(II)

erhält;

b) das O-Trimethylsilylcyanohydrin der Formel (II) mit einer Säure in einem inerten Medium hydrolisiert, wobei man das Cyanohydrin der Formel (III):

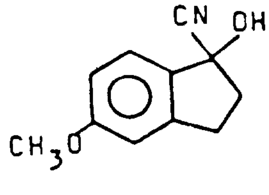

(III)

erhält;

c) das Cyanohydrin der Formel (III) mit Zinn-II-Chlorid in saurem Medium behandelt, wobei man die Säure der Formel (IV):

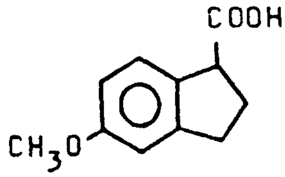

(IV)

erhält;

d) die Säure der Formel (IV) demethyliert, wobei man die Säure der Formel (V):

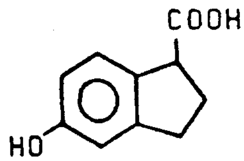

(V)

erhält;

e) die Säure der Formel (V) mit einem aliphatischen Alkohol, der bis zu 4 Kohlenstoffatome und die allgemeine Formel ROH (V') aufweist, in Anwesenheit einer Säure als Katalysator verestert, wobei man einen Ester der allgemeinen Formel (VI'):

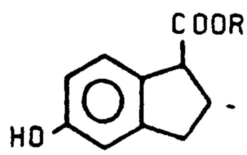

(VI')

worin R die angegebenen Bedeutungen besitzt, erhält;

f) einen Ester der allgemeinen Formel (VI') mit X-$(CH_2)_n$-Y, worin X und Y unabhängig für Chlor, Brom, Jod oder $OSO_2R_2$'stehen, worin $R_2$' für Phenyl, p-Methylphenyl oder Methyl steht, in Anwesenheit einer Base und in einem nichtpolaren Medium alkyliert, wobei man einen Ester der allgemeinen Formel (VII');

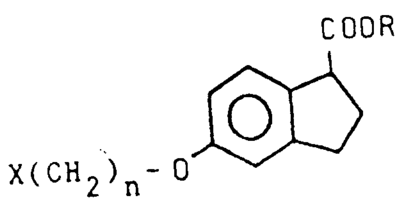

(VII')

worin R, X und n die angegebenen Bedeutungen besitzen, erhält;

g) einen Ester der allgemeinen Formel (VII') mit Imidazol in Lösungin einemN,N-substituierten linearen Amid und in Anwesenheit eines basischen Coadjuvans zur Reaktion bringt, wobei man einen Ester der allgemeinen Formel (VIII'):

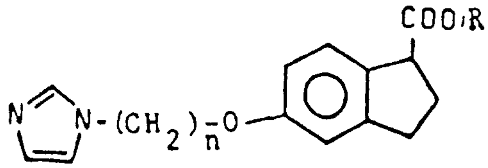

VIII'

worin R und n die angegebenen Bedeutungen besitzen, erhält;
h) einen Ester der allgemeinen Formel (VIII') hydrolysiert.

**2.** Verfahren nach Anspruch 1, wobei es sich bei der Lewissäure in Stufe a) um Bortrifluorid-Ätherat handelt.

**3.** Verfahren nach Anspruch 1 oder 2, wobei es sich bei der Säure in Stufe b) um eine Mineralsäure, vorzugsweise Chlorwasserstoffsäure, handelt.

**4.** Verfahren nach einem der vorhergehenden Ansprüche, wobei es sich bei dem inerten Medium in Stufe b) um ein ätherisches Medium, vorzugsweise Tetrahydrofuran, handelt.

**5.** Verfahren nach einem der hervorgehenden Ansprüche, wobei es sich bei dem sauren Medium in Stufe c) um eine Mischung aus konzentrierter Salzsäure und Eisessig handelt.

**6.** Verfahren nach einem der vorhergehenden Ansprüche, wobei man die Stufe d) in einem stark sauren Medium, vorzugsweise in konzentrierter Bromwasserstoffsäure, durchführt.

**7.** Verfahren nach einem der vorhergehenden Ansprüche, wobei es sich bei dem Katalysator in Stufe e) um eine Sulfonsäure, vorzugsweise p-Toluolsulfonsäure, handelt.

**8.** Verfahren nach einem der vorhergehenden Ansprüche, wobei in Stufe f) X für Chlor und Y für $OSO_2R''$, vorzugsweise p-Toluolsulfonyl, steht.

**9.** Verfahren nach einem der vorhergehenden Ansprüche, wobei es sich bei der Base in Stufe f) um ein Alkali- oder Erdalkalimetallcarbonat oder -bicarbonat, vorzugsweise Kaliumcarbonat, handelt.

**10.** Verfahren nach einem der vorhergehenden Ansprüche, wobei es sich bei dem nicht-plolaren Medium in Stufe f) um ein aliphatisches Keton, vorzugsweise Methylethylketon, handelt.

**11.** Verfahren nach einem der vorhergehenden Ansprüche, wobei es sich bei dem N,N-disubstituierten linearen Amid in Stufe g) um Dimethylformamid handelt.

**12.** Verfahren nach einem der vorhergehenden Ansprüche, wobei es sich bei dem basischen Co-Adjuvans in Stufe g) um Natriumhydrid handelt.

**13.** Verfahren nach einem der vorhergehenden Ansprüche, wobei es sich bei der Mineralsäure in Stufe h) um Salzsäure handelt.

**14.** Verfahren nach Anspruch 2, wobei R in den allgemeinen Formeln V', VI', VII' und VIII' für Methyl steht.

**15.** Verfahren nach einem der vorhergehenden Ansprüche, wobei man eine Verbindung der allgemeinen Formel (I) erhält, in der n für 2 steht.

**16.** Verbindungen der allgemeinen Formel:

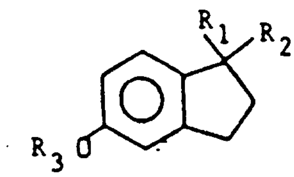

(III)

worin

a) $R_1$ für Cyano, $R_2$ für Trimethylsilyloxy oder Hydroxy und $R_3$ für Methyl stehen;

b) $R_1$ für COOH steht;
$R_2$ für H steht und $R_3$ für Wasserstoff, Methyl oder $X(CH_2)_n$-steht, worin n für 1 bis 4 steht und X für Chlor, Brom, Jod oder $OSO_2R_2$' steht, wobei $R_2$' für Phenyl, p-Methylphenyl oder Methyl steht; oder

c) $R_1$ für COOR steht, worin R für $C_1$ - $C_4$-Alkyl steht,
$R_2$ für H steht und $R_3$ für Wasserstoff, Methyl oder $X(CH_2)_n$-steht, wobei n für 1 bis 4 steht und X für Chlor, Brom, Jod, Imidazol-1-yl oder $OSO_2R_2$' steht und $R_2$' für Phenyl, p-Methylphenyl oder Methyl steht.

## Revendications

**1.** Procédé pour la préparation de dérivés de type 2,3-dihydro-1H-imidazolylalcoxyindène de formule (I):

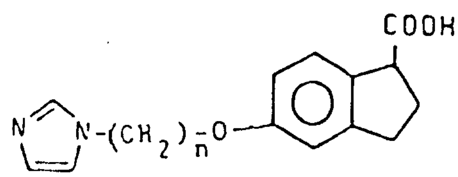

(I)

dans laquelle n va de 1 à 4
et de leurs sels pharmaceutiquement acceptables,
caractérisé en ce que

a) on fait réagir de la 5-méthoxy-1-indanone avec du cyanure de triméthylsilyle, en présence d'un acide de Lewis en tant que catalyseur, à une température élevée, pour obtenir l'O-triméthylsilylcyanohydrine de formule (II ):

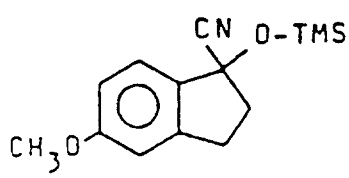

(II)

b) on hydrolyse l'O-triméthylsilylcyanohydrine de formule (II) avec un acide, dans un milieu inerte, pour obtenir la cyanohydrine de formule (III):

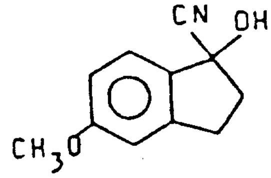

(III)

c) on traite la cyanohydrine de formule (III) par du chlorure stanneux, dans un milieu acide, pour obtenir l'acide de formule (IV):

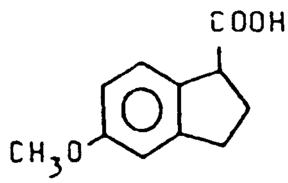

(IV)

d) on soumet à une déméthylation l'acide de formule (IV), pour obtenir l'acide de formule (V):

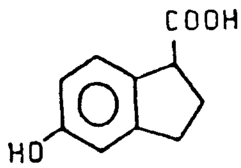

(V)

e) on estérifie l'acide de formule (V) avec un alcool aliphatique ayant jusqu'à 4 atomes de carbone, de formule générale ROH (V'), en présence d'un acide en tant que catalyseur, pour obtenir un ester de formule générale (VI'):

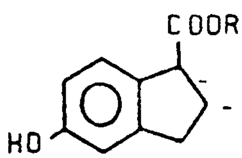

(VI')

dans laquelle R est tel que défini plus haut;

f) on soumet un ester de formule générale (VI') à une alkylation avec un composé de formule $X-(CH_2)_n-Y$, X et Y pouvant indépendamment être un atome de chlore, de brome ou d'iode ou un groupe $OSO_2R'_2$ dans lequel $R'_2$ est le radical phényle, p-méthylphényle ou méthyle, en présence d'une base et dans un milieu non polaire, pour obtenir un ester de formule générale (VII'):

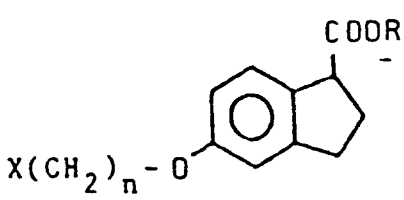

(VII')

dans laquelle R, X et n sont tels que définis plus haut;

g) on fait réagir un ester de formule générale (VII') avec de l'imidazole en solution dans un amide linéaire N,N-substitué, et en présence d'un co-adjuvant basique, pour obtenir un ester de formule générale (VIII'):

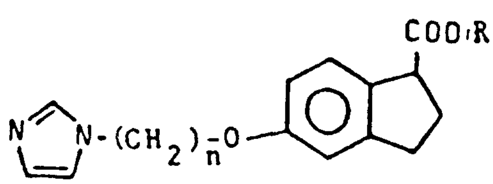

VIII'

dans laquelle R et n sont tels que définis plus haut;
h) on hydrolyse un ester de formule générale (VIII').

**2.** Procédé selon la revendication 1, dans lequel ledit acide de Lewis, dans l'étape a), est l'éthérate de trifluorure de bore.

**3.** Procédé selon la revendication 1 ou 2, dans lequel ledit acide, dans l'étape b), est un acide minéral, de préférence l'acide chlorhydrique.

**4.** Procédé selon l'une quelconque des revendications précédentes, dans lequel ledit milieu inerte, dans l'étape b), est un milieu de type éther, de préférence le tétrahydrofuranne.

**5.** Procédé selon l'une quelconque des revendications précédentes, dans lequel ledit milieu acide, dans l'étape c), est un mélange d'acide chlorhydrique concentré et d'acide acétique glacial.

**6.** Procédé selon l'une quelconque des revendications précédentes, dans lequel l'étape d) est effectuée dans un milieu constitué d'un acide fort, de préférence dans de l'acide bromhydrique concentré.

**7.** Procédé selon l'une quelconque des revendications précédentes, dans lequel le catalyseur, dans l'étape e), est un acide sulfonique, de préférence l'acide p-toluènesulfonique.

**8.** Procédé selon l'une quelconque des revendications précédentes, dans lequel, dans l'étape f), X est un atome de chlore et Y est un groupe $OSO_2R''$, de préférence le groupe p-toluènesulfonyle.

**9.** Procédé selon l'une quelconque des revendications précédentes, dans lequel ladite base, dans l'étape f), est un carbonate ou bicarbonate alcalin ou alcalino-terreux, de préférence le carbonate de potassium.

**10.** Procédé selon l'une quelconque des revendications précédentes, dans lequel ledit solvant non polaire, dans l'étape f), est une cétone aliphatique, de préférence la méthyléthylcétone.

**11.** Procédé selon l'une quelconque des revendications précédentes, dans lequel ledit amide linéaire N,N-disubstitué, dans l'étape g), est le diméthylformamide.

**12.** Procédé selon l'une quelconque des revendications précédentes, dans lequel ledit co-adjuvant basique, dans l'étape g), est l'hydrure de sodium.

**13.** Procédé selon l'une quelconque des revendications précédentes, dans lequel ledit acide minéral, dans l'étape h), est l'acide chlorhydrique.

**14.** Procédé selon la revendication 2, dans lequel R, dans les formules générales V', VI', VII' et VIII', est le groupe méthyle.

**15.** Procédé selon l'une quelconque des revendications précédentes, dans lequel on obtient un composé de formule générale (I) dans lequel n est 2.

**16.** Composés de formule générale

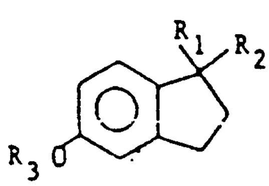

(III)

dans laquelle

a) $R_1$ est est le groupe cyano, $R_2$ est le groupe triméthylsilyloxy ou hydroxy, et $R_3$ est le groupe méthyle;
b) $R_1$ est COOH;
$R_2$ est H et $R_3$ est un atome d'hydrogène ou le groupe méthyle ou un groupe $X(CH_2)_n$- dans lequel n va de 1 à 4 et X est un atome de chlore, de brome ou d'iode, ou un groupe $OSO_2R'_2$ dans lequel $R'_2$ est le groupe phényle, p-méthylphényle ou méthyle; ou
c) $R_1$ est un groupe COOR dans lequel R est un radical alkyle en $C_1$-$C_4$; $R_2$ est H et $R_3$ est un atome d'hydrogène, le groupe méthyle ou un groupe $X(CH_2)_n$-, n allant de 1 à 4 et X étant un atome de chlore, de brome ou d'iode ou le groupe imidazol-1-yle ou un groupe $OSO_2R'_2$, et $R'_2$ est le radical phényle, p-méthylphényle ou méthyle.